(19) Europäisches Patentamt
European Patent Office
Office européen des brevets

(11) Veröffentlichungsnummer: **0 009 633**
**B1**

# (12) EUROPÄISCHE PATENTSCHRIFT

(45) Veröffentlichungstag der Patentschrift: **22.09.82**

(51) Int. Cl.³: **C 07 D211/46**, C 07 D211/60, A 61 K 31/445, A 23 K 1/16

(21) Anmeldenummer: **79103241.0**

(22) Anmeldetag: **03.09.79**
**Die Akte enthält technische Angaben, die nach dem Eingang der Anmeldung eingereicht wurden und die nicht in dieser Patentschrift enthalten sind.**

(54) **3,4,5-Trihydroxypiperidin-Derivate, Verfahren zu ihrer Herstellung sowie diese enthaltende Arzneimittel und Tierfuttermittel.**

(30) Priorität: **09.09.78 DE 2839309**

(43) Veröffentlichungstag der Anmeldung: **16.04.80 (Patentblatt 80/08)**

(45) Bekanntmachung des Hinweises auf die Patenterteilung: **22.09.82 Patentblatt 82/38**

(84) Benannte Vertragsstaaten: **AT BE CH DE FR GB IT NL SE**

(56) Entgegenhaltungen: **EP - A - 0 000 947**

(73) Patentinhaber: **BAYER AG**
**Zentralbereich Patente, Marken und Lizenzen**
**D-5090 Leverkusen 1, Bayerwerk (DE)**

(72) Erfinder: **Stoltefuss, Jürgen, Ing.-grad.**
**Parkstrasse 20**
**D-5657 Haan 2 (DE)**
Erfinder: **Müller, Lutz, Dr.**
**Kronprinzenallee 111**
**D-5600 Wuppertal (DE)**
Erfinder: **Puls, Walter, Dr.**
**In den Birken 75**
**D-5600 Wuppertal 1 (DE)**
Erfinder: **Sitt, Rüdiger, Dr.**
**Claudiusweg 9**
**D-5600 Wuppertal (DE)**

**EP 0 009 633 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# 3,4,5-Trihydroxypiperidin-Derivate, Verfahren zu ihrer Herstellung sowie diese enthaltende Arzneimittel und Tierfuttermittel

Die vorliegende Erfindung betrifft neue Derivate des 3,4,5-Trihydroxypiperidins, Verfahren zu ihrer Herstellung sowie diese enthaltende Arzneimittel, beispielsweise Mittel gegen Prädiabetes, Gastritis, Obstipation, Infektionen des Gastro-Intestinal-Traktes, Meteorismus, Flatulenz, Karies, Atherosklerose, Hypertension und insbesondere gegen Diabetes, Hyperlipämie und Adipositas sowie Tierfuttermittel zur Beeinflussung des Fleisch-Fett-Verhältnisses zugunsten des Fleischanteils.

Die erfindungsgemäßen Verbindungen entsprechen der allgemeinen Formel I

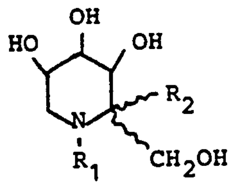

(I)

worin

$R_1$ Wasserstoff oder einen gegebenenfalls durch Halogen, Hydroxy oder $C_1$-$C_4$-Alkoxy substituierten geradkettigen, verzweigten, gesättigten oder ungesättigten aliphatischen Kohlenwasserstoffrest mit bis zu 18 C-Atomen darstellt und

$R_2$ $-CH_2NR_3R_4$, $-CH_2-NR_3-CO-R_4$, $-CH_2-NR_3-CONR_4R_5$, $-CH_2-NR_3-SO_2R_4$, $-CH_2-NR_3-SO_2-NR_4-R_5$, $-CH_2-NR_3-CS-R_4$, $-CH_2-NR_3-CS-NR_4R_5$, $-COOR_3$, $-CO-NR_3R_4$, $-CH_2-OR_3$, $-CH_2-NH-COOR_4$ oder $-CH_2-NH-COSR_4$,

$R_3$, $R_4$ und $R_5$ Wasserstoff, einen gegebenenfalls durch hydroxy, $C_1$-$C_4$-Alkoxy, $C_1$-$C_4$-Alkoxycarbonyl, Carboxy, Amino, Di-$C_1$-$C_4$-Alkylamino oder Cyan substituierten aliphatischen Kohlenwasserstoffrest mit bis zu 18 C-Atomen, eine $C_5$-$C_{12}$-Cycloalkylgruppe, Benzyl oder einen Phenylrest der gegebenenfalls durch $C_1$-$C_4$-Alkyl, $C_1$-$C_4$-Alkoxy, Halogen, Nitro, $CF_3$; Carboxy, Alkoxycarbonyl, Amino oder den $-CH=CH-COOC_2H_5$-Rest substituiert ist,

bedeuten.

Es wurde gefunden, daß die neuen Verbindungen der Formel I potente Inhibitoren für α-Glucosidasen, insbesondere für Disaccharidasen sind. Daher sind die neuen Verbindungen wertvolle Mittel zur Beeinflussung einer Vielzahl von Stoffwechselvorgängen und bereichern somit den Arzneimittelschatz. Gegenüber dem aus der DT-OS 2 656 602 bekannten 2-Hydroxy-methyl-3,4,5-trihydroxypiperidin weisen die neuen Verbindungen vorteilhafte therapeutische Eigenschaften auf.

Die erfindungsgemäßen Verbindungen werden erhalten, indem man 2,6-Imino-2-hydroxymethyl-2,6-didesoxy-L-ido (L-gulo)-hexonsäurenitril der Formel III

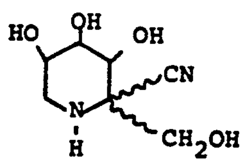

(III)

entweder

a) katalytisch zur Verbindung der Formel IV

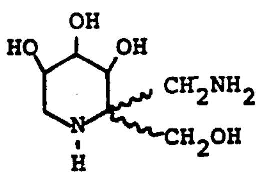

(IV)

hydriert und anschließend gegebenenfalls die primäre Aminogruppe alkyliert, acyliert, sulfonyliert oder mit Isocyanaten oder Isothiocyanaten umsetzt und anschließend gegebenenfalls am sekundären Stickstoffatom alkyliert oder

b) die Verbindung der Formel III zur Verbindung der Formel V

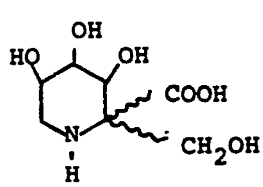

(V)

verseift und anschließend gegebenenfalls verestert und die Ester gegebenenfalls entweder mit Aminen zu Amiden umsetzt oder gegebenenfalls mit Wasserstoff-Donor-Reduktionsmitteln zu Verbindungen der Formel VI

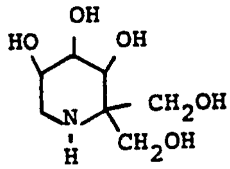

(VI)

reduziert und alle auf diesem Wege entstandenen Verbindungen gegebenenfalls am sekundären Stickstoffatom alkyliert, oder indem man Verbindungen der Formel VII

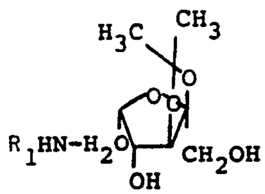

(VII)

worin $R_1$ die oben angegebene Bedeutung hat, mit Mineralsäuren zu Verbindungen der Formel VIII

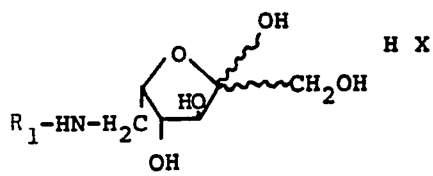

(VIII)

deblockiert und diese dann direkt in Lösung oder nach Isolierung mit Cyaniden zu Verbindungen der Formel IX

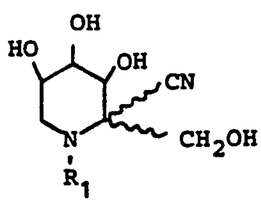

(IX)

umsetzt und gegebenenfalls am sekundären Stickstoffatom alkyliert.

Die verwendeten Ausgangsverbindungen sind zum größten Teil bekannt oder können nach an sich bekannten Methoden leicht hergestellt werden (vgl. H. Paulsen, J. Sangster und K. Heyns, Chem. Ber. *100*, 802-815 (1967)).

Als Wasserstoff-Donor-Reduktionsmittel können z.B. katalytischer Wasserstoff, Alkalimetallborhydride, Alkalimetallcycanoborhydride, Dialkylaminoborane oder Ameisensäure verwendet werden. Bei der reduktiven Aminierung zu N-Alkylaminen verwendet man vorzugsweise Natriumcyanoborhydrid, in der Hydrierung der Nitrilgruppe zur Aminomethylgruppe vorzugsweise Wasserstoff in Gegenwart von Raney-Nickel oder in Gegenwart von Edelmetallkatalysatoren. Die Hydrierung wird im allgemeinen bei Drucken von 1 bis 150 bar Wasserstoff und Temperaturen zwischen 20 und 150 °C durchgeführt, als Lösungsmittel werden dabei protische, polare Lösungsmittel, insbesondere Wasser und Alkohole, bevorzugt.

Die in dem Verfahren eingesetzten Aldehyde, Ketone, Carbonsäurechloride, Sulfonsäurechloride, Sulfamoylchloride, Isocyanate und Isothiocyanate sind zum größten Teil bekannt. Neue Verbindungen dieser Gruppen können nach konventionellen Methoden hergestellt werden. Beispielsweise seien Formaldehyd, Acetaldehyd, Propionaldehyd, Butyraldehyd, 1-Pentanal, 1-Hexanal, Aceton, 4-Heptanon, Cyclohexanon, Cyclopentanon, Benzaldehy, Acetylchlorid, Propionylchlorid, Buttersäurechlorid, Benzoylchlorid, 4-Methoxybenzoylchlorid, 3-Methylbenzoylchlorid, Methansulfonsäurechlorid, Benzolsulfonsäurechlorid, Dimethylcarbamoylchlorid, Äthylisocyanat, Phenylisocyanat, 4-Chlorphenylisocyanat, Methylisocyanat, 4-Toluolsulfonsäurechlorid, Dimethylsulfamoylchlorid, Methylisothiocyanat, Butylisocyanat, Hexylisocyanat, Allylisothiocyanat und Phenylisothiocyanat genannt.

Als neue Wirkstoffe seien im einzelnen aufgeführt

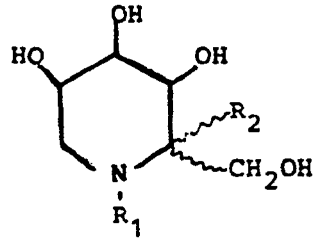

| $R_1$ | $R_2$ |
|---|---|
| H | $-CH_2-NH_2$ |
| H | $-CH_2-NH-CO-$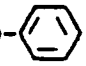 |
| $-CH_3$ | $-CH_2-NH-CO-$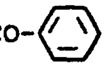 |
| $-C_2H_5$ | $-CH_2-NH-CO-$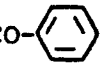 |
| $-C_3H_7$ | $-CH_2-NH-CO-$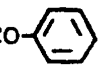 |
| $-C_4H_9$ | $-CH_2-NH-CO-$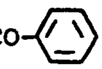 |
| $-C_5H_{11}$ | $-CH_2-NH-CO-$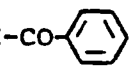 |
| $-C_6H_{13}$ | $-CH_2-NH-CO-$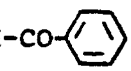 |
| $-C_7H_{15}$ | $-CH_2-NH-CO-$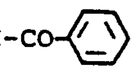 |
| $-C_8H_{17}$ | $-CH_2-NH-CO-$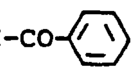 |
| $-C_{10}H_{21}$ | $-CH_2-NH-CO-$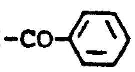 |
| $-C_{12}H_{25}$ | $-CH_2-NH-CO-$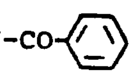 |
| $-C_{14}-H_{29}$ | $-CH_2-NH-CO-$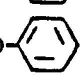 |
| H | $-CH_2-NH-CO-$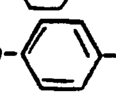$-NO_2$ |
| $-C_9H_{19}$ | $-CH_2-NH-CO-$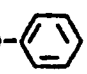$-NO_2$ |
| H | $-CH_2-NH-CO-$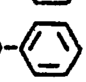$-CH_3$ |
| $-CH_3$ | $-CH_2-NH-CO-$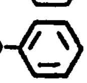$-CH_3$ |
| $-C_8H_{17}$ | $-CH_2-NH-CO-$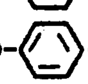$-CH_3$ |
| -H | $-CH_2-NH-CO-$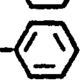$-OCH_3$ |
| $-C_2H_5$ | $-CH_2-NH-CO-$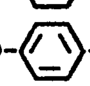$-OCH_3$ |
| -H | $-CH_2-NH-SO_2-$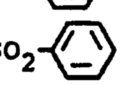$-CH_3$ |

(Fortsetzung)

| $R_1$ | $R_2$ |
|---|---|
| $-CH_3$ | $-CH_2-NH-SO_2-$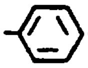$-CH_3$ |
| $-C_2H_5$ | $-CH_2-NH-SO_2-$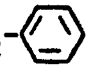$-CH_3$ |
| $-C_4H_9$ | $-CH_2-NH-SO_2-$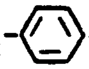$-CH_3$ |
| $-C_7H_{15}$ | $-CH_2-NH-SO_2-$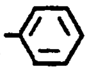$-CH_3$ |
| $-C_9H_{19}$ | $-CH_2-NH-SO_2-$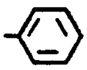$-CH_3$ |
| $-C_{11}H_{23}$ | $-CH_2-NH-SO_2-$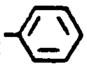$-CH_3$ |
| H | $-CH_2-NH-SO_2-$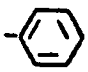 |
| $-CH_3$ | $-CH_2-NH-SO_2-$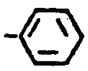 |
| $C_8H_{17}$ | $-CH_2-NH-SO_2-$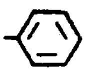 |
| H | $-CH_2-NH-SO_2-$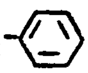$-Cl$ |
| $CH_3$ | $-CH_2-NH-SO_2-$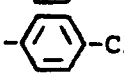$-Cl$ |
| H | $-CH_2-NH-CO-NH-CH_3$ |
| $-CH_3$ | $-CH_2-NH-CO-NH-CH_3$ |
| $-C_6H_{13}$ | $-CH_2-NH-CO-NH-CH_3$ |
| $-CH_2-CH=CH_2$ | $-CH_2-NH-CO-$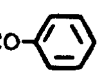 |
| $-CH_2-CH=CH_2$ | $-CH_2-NH-SO_2-$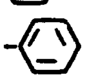$-CH_3$ |
| $-CH_2-CH=CH_2$ | $-CH_2-NH-CO-NH-CH_3$ |
| $-H$ | $-CH_2-NH-CO-NH-$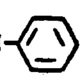 |
| $-CH_3$ | $-CH_2-NH-CO-NH-$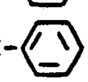 |
| $-C_7H_{15}$ | $-CH_2-NH-CO-NH-$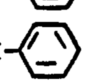 |
| H | $-CH_2-NH-CO-CH_3$ |
| $-CH_3$ | $-CH_2-NH-CO-CH_3$ |
| $-C_2H_5$ | $-CH_2-NH-CO-CH_3$ |
| $-C_8H_{17}$ | $-CH_2-NH-CO-CH_3$ |
| H | $-CH_2-NH-CO-C_2H_5$ |

(Fortsetzung)

| $R_1$ | $R_2$ |
|---|---|
| $-CH_3$ | $-CH_2-NH-CO-C_2H_5$ |
| H | $-CH_2-NH-CO-C_3H_7$ |
| $-C_2H_5$ | $-CH_2-NH-CO-C_3H_7$ |
| H | $-CH_2-NH-CO-C_6H_{13}$ |
| $-CH_3$ | $-CH_2-NH-CO-C_6H_{13}$ |
| H | $-CH_2-NH-CO-C_8H_{17}$ |
| $-C_6H_{13}$ | $-CH_2-NH-CO-C_8H_{17}$ |
| H | $-CH_2-NH-CO-C_{17}H_{35}$ |
| $-CH-C\equiv CH$ | $-CH_2-NH-CO-C_{17}H_{35}$ |
| H | $-CH_2-N-CO-$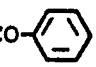<br>$CH_3$ |
| $-CH_3$ | $-CH_2-N-CO-$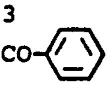<br>$CH_3$ |
| H | $-CH_2-NH-CS-NH-CH_3$ |
| $CH_3$ | $-CH_2-NH-CS-NH-CH_3$ |
| H | $-CH_2-NH-CS-NH-$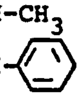 |
| $-C_4H_9$ | $-CH_2-NH-CS-NH-$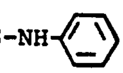 |
| H | $-CH_2-NH-COOC_2H_5$ |
| $CH_3$ | $-CH_2-NH-COOC_2H_5$ |
| H | $-CH_2-NH-CO-SC_2H_5$ |
| $CH_3$ | $-CH_2-NH-COSC_2H_5$ |
| H | $-CH_2-NH-SO_2-CH_3$ |
| $CH_3$ | $-CH_2-NH-SO_2-CH_3$ |
| H | $-CH_2-NH-SO_2-N(CH_3)_2$ |
| $C_2H_5$ | $-CH_2-NH-SO_2-N(CH_3)_2$ |
| H | $-CH_2-NH-C_4H_9$ |
| $CH_3$ | $-CH_2-NH-C_8H_{19}$ |
| $CH_3$ | $-CH_2-N(CH_3)_2$ |
| H | $-COOH$ |
| H | $-COOC_2H_5$ |
| $CH_3$ | $-COOH$ |
| $C_8H_{17}$ | $-COOH$ |

(Fortsetzung)

| $R_1$ | $R_2$ |
|---|---|
| $CH_3$ | $-COOC_2H_5$ |
| H | $-CONH_2$ |
| $CH_3$ | $-CONH_2$ |
| H | $-CH_2-NH-SO_2C_3H_7$ |
| H | $-CH_2OH$ |
| $CH_3$ | $-CH_2OH$ |
| H | $-CH_2-O-COCH_3$ |
| $CH_3$ | $-CH_2-O-COCH_3$ |
| H | $-COOC_8H_{17}$ |
| $-CH_3$ | $-COOC_8H_{17}$ |
| $-C_6H_{13}$ | $-COOC_8H_{17}$ |
| $HO-CH_2-CH_2-$ | $-CH_2-NH-CO-$ 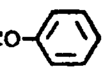 |
| $-CH_2-C{\equiv}CH$ | $-CH_2-NH-CO-$ 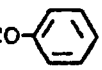 |
| $-CH_3$ | $-CH_2-NH-CH_2-CH_2-CN$ |

Die erfindungsgemäßen Inhibitoren eignen sich als Therapeutica für folgende Indikationen: Prädiabetes, Gastritis, Obstipation, Infektionen des Gastrointestinal-Traktes, Meteorismus, Flatulenz, Karies, Atherosklerose, Hypertension und besonders Adipositas, Diabetes und Hyperlipämie.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, daß es sich um Kombinationen der erfindungsgemäßen Inhibitoren untereinander oder um Kombinationen der erfindungsgemäßen Inhibitoren mit bereits bekannten handelt. So kann es beispielsweise zweckmäßig sein, erfindungsgemäße Saccharase-inhibitoren mit bereits bekannten Amylase-Inhibitoren zu kombinieren.

Vorteilhaft sind in manchen Fällen auch Kombinationen der erfindungsgemäßen Inhibitoren mit bekannten oralen Antidiabetica (β-cytotrope Sulfonylharnstoffderivate und/oder blutzuckerwirksame Biguanide) sowie mit blutlipidsenkenden Wirkstoffen wie z.B. Clofibrat, Nicotinsäure, Cholestyramin und anderen.

Die Verbindungen können ohne Verdünnung, z.B. als Pulver oder in einer Gelatinehülle oder in Kombination mit einem Trägerstoff in einer pharmazeutischen Zusammensetzung appliziert werden.

Die unter Verwendung der erfindungsgemäßen Wirkstoffe hergestellten Nahrungsmittel eignen sich sowohl zur Diät bei Patienten, die an Stoffwechselstörungen leiden als auch zur Ernährung gesunder Personen im Sinne einer Stoffwechselstörungen vorbeugenden Ernährungsweise.

Die erfindungsgemäßen Inhibitoren weisen weiterhin die Eigenschaft auf, in Tieren das Verhältnis des Anteils an unerwünschtem Fett zum Anteil des erwünschten fettarmen Fleisches (mageres Fleisch) zugunsten des mageren Fleisches in hohem Maße zu beeinflussen. Dies ist von besonderer Bedeutung für die Aufzucht und Haltung von landwirtschaftlichen Nutztieren, z.B. in der Schweinemast, aber auch von erheblicher Bedeutung für die Aufzucht und Haltung von sonstigen Nutztieren und Ziertieren. Die Verwendung der Inhibitoren kann weiterhin zu einer erheblichen Rationalisierung der Fütterung der Tiere führen, sowohl zeitlich, mengenmäßig wie auch qualitätsmäßig. Da sie eine gewisse Verzögerung der Verdauung bewirken, wird die Verweildauer der Nährstoffe im Verdauungstrakt verlängert, wodurch eine mit weniger Aufwand verbundene ad libitum-Fütterung ermöglicht wird. Weiterhin ergibt sich bei der Verwendung der erfindungsgemäßen Inhibitoren in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowie der Einsparung von Futtereiweiß verwendet werden.

Die Wirksamkeit der Wirkstoffe ist hierbei weitgehend unabhängig von der Art und dem Geschlecht der Tiere. Besonders wertvoll erweisen sich die Wirkstoffe bei Tierarten, die überhaupt oder in bestimmten Lebensabschnitten zu stärkerer Fetteinlagerung neigen.

Als Tiere, bei denen die Inhibitoren zur Reduzierung des Fettansatzes und/oder zur Einsparung von Futtereiweiß eingesetzt werden können, seien beispielsweise folgende Nutz- und Ziertiere genannt: Warmblüter wie Rinder, Schweine, Pferde, Schafe, Ziegen, Katzen, Hunde, Kaninchen, Pelztiere, z.B. Nerze, Chinchilla, andere Ziertiere, z.B. Meerschweinchen und Hamster, Labor- und Zootiere, z.B. Ratten, Mäuse, Affen usw.; Geflügel, z.B. Broiler, Hühner, Gänse, Enten, Truthähne, Tauben, Papageien und Kanarienvögel und Kaltblüter, wie Fische, z.B. Karpfen und Reptilien, z.B. Schlangen.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,1 mg bis 1,0 g, insbesondere 1 bis 100 mg/kg Futter pro Tag. Die Dauer der Verabreichung kann von wenigen Stunden oder Tagen bis zu mehreren Jahren betragen. Die passende Menge Wirkstoff sowie die passende Dauer der Verabreichung stehen in engem Zusammenhang mit dem Fütterungsziel. Sie hängen insbesondere von der Art, dem Alter, dem Geschlecht, dem Gesundheitszustand und der Art der Haltung der Tiere ab und sind durch jeden Fachmann leicht zu ermitteln.

Beispiel für die Zusammensetzung eines fertigen Mischfutters für Geflügel, das einen erfindungsgemäßen Wirkstoff enthält:

200 g Weizen, 340 g Mais, 360,3 g Sojaschrot, 60 g Rindertalg, 15 g Dicalciumphosphat, 10 g Calciumcarbonat, 4 g jodiertes Kochsalz, 7,5 g Vitamin-Mineral-Mischung und 3,2 g Wirkstoff-Premix ergeben nach sorgfältigem Mischen 1 kg Futter.

Die Vitamin-Mineral-Mischung besteht aus:

6 000 I.E. Vitamin A, 1 000 I.E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 g $MnSO_4 \times H_2O$, 140 mg $ZnSO_4 \times 7H_2O$, 100 mg $FeSO_4 \times 7H_2O$ und 20 mg $CuSO_4 \times 5H_2O$.

Der Wirkstoff-Premix enthält einen erfindungsgemäßen Wirkstoff in der gewünschten Menge, z.B. 1 600 mg und zusätzlich 1 g DL-Methionin sowie so viel Sojabohnenmehl, daß 3,2 g Premix entstehen.

Beispiel für die Zusammensetzung eines Schweinemischfutters, das einen Wirkstoff der Formel I enthält:

630 g Futtergetreideschrot (zusammengesetzt aus 200 g Mais-, 150 g Gerste-, 150 g Hafer- und 130 g Weizenschrot), 80 g Fischmehl, 60 g Sojaschrot, 58,8 g Tapiokamehl, 38 g Bierhefe, 50 g Vitamin-Mineral-Mischung für Schweine (Zusammensetzung, z.B. wie beim Kükenfutter), 30 g Leinkuchenmehl, 30 g Maiskleberfutter, 10 g Sojaöl, 10 g Zuckerrohrmelasse und 2 g Wirkstoff-Premix (Zusammensetzung z.B. beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Die Inhibitoren können einzeln oder aber auch in beliebigen Mischungen untereinander verwendet werden.

Saccharase-Inhibitionstest in vitro

Der Saccharase-Inhibitionstest in vitro ermöglicht die Bestimmung der enzyminhibitorischen Aktivität einer Substanz durch den Vergleich der Aktivität des solubilisierten intestinalen Disaccharidasen-Komplexes in Gegenwart bzw. in Abwesenheit (sog. 100 %-Wert) des Inhibitors. Als Substrat, welches die Spezifität des Inhibitionstestes bestimmt, dient dabei eine praktisch Glucose-freie Saccharose (Glucose < 100 ppm); die Enzymaktivitätsbestimmung basiert auf der spektrophotometrischen Bestimmung freigesetzter Glucose mittels Glucose-Dehydrogenase und Nicotinamid-adenin-dinucleotid als Cofaktor.

Eine Saccharase-Inhibitor-Einheit (SIE) ist definiert als diejenige inhibitorische Aktivität, welche in einem definierten Testansatz eine vorgegebene saccharolytische Aktivität um eine Einheit (Saccharase-Einheit = SE) reduziert; die Saccharase-Einheit ist dabei als diejenige Enzymaktivität definiert, welche unter vorgegebenen Bedingungen ein µmol Saccharose pro Minute spaltet und damit zur Freisetzung von je ein µmol Glucose, welche im Test bestimmt wird, und Fructose, welche im Test nicht erfaßt wird, führt.

Der intestinale Disaccharidasen-Komplex wird aus Schweinedünndarm-Mucosa durch tryptische Verdauung, Fällung aus 66 % Äthanol bei − 20 °C, Aufnehmen des Präcipitates in 100 mM Phosphat-Puffer, pH 7,0 und abschließende Dialyse gegen denselben Puffer gewonnen.

10 µl einer Probelösung, die so angesetzt ist, daß die Extinktion des Testansatzes mindestens 10 %, jedoch nicht mehr als 25 % unter der des 100 %-Wertes liegt, werden mit 100 µl einer Verdünnung des intestinalen Disaccharidasen-Komplexes in 0,1 M Maleinat-Puffer, pH 6,25, versetzt und für 10 Minuten bei 37 °C vorinkubiert. Die Verdünnung des Disaccharidasen-Komplexes ist auf eine Aktivität von 0,1 SE/ml einzustellen.

Anschließend wird die saccharolytische Reaktion durch Zugabe von 100 µl einer 0,4 M Lösung von Saccharose (« SERVA 35579 ») in 0,1 M Maleinat-Puffer, pH 6,25 gestartet und nach einer Inkubationsdauer von 20 Minuten bei 37 °C durch die Zugabe von 1 ml Glucose-Dehydrogenase-Reagenz (1 Fläschchen Glucose-Dehydrogenase-Mutarotase-Gemisch lyophiliert (« MERCK 14053 ») und 331,7 mg β-Nicotinamid-adenin-dinucleotid (freie Säure, « BOEHRINGER » Reinheitsgrad I) in 250 ml 0,5 M Tris-Puffer, pH 7,6 gelöst) abgestoppt. Zum Nachweis der Glucose wird 30 Minuten bei 37 °C inkubiert und schließlich bei 340 nm gegen einen Reagenzienblank (mit Enzym, jedoch ohne Saccharose) photometriert.

Die Berechnung der Hemmaktivität von Inhibitoren ist dadurch erschwert, daß schon geringfügige Änderungen im Testsystem, beispielsweise ein geringfügig von Bestimmung zu Bestimmung variierender 100 %-Wert, von nicht mehr zu vernachlässigendem Einfluß auf das Testergebnis sind. Man umgeht diese Schwierigkeiten, indem man bei jeder Bestimmung einen Standard mitlaufen läßt ; als Standard dient ein Saccharase-Inhibitor der Formel $C_{25}H_{43}O_{18}N$, welcher eine spezifische Hemmaktivität von 77 700 SIE/g aufweist und bei eingesetzten Mengen von 10 bis 20 ng im Test zu einer Hemmung von oben spezifizierter Größenordnung führt. Bei Kenntnis der Differenz der Extinktionen bei 340 nm von 100 %-Wert und durch Standard gehemmtem Ansatz läßt sich aus der Extinktionsdifferenz von 100 %-Wert und durch die Probelösung gehemmtem Ansatz unter Berücksichtitung der eingesetzten Menge an Inhibitor in bekannter Weise dessen spezifische Hemmaktivität errechnen, ausgedrückt in Saccharase-Inhibitor-Einheiten pro Gramm (SIE/g).

## Beispiel 1

### Herstellung des Ausgangsproduktes

2,6-Imino-2-hydroxymethyl-2,6-didesoxy-L-ido (L-gulo) hexonsäurenitril

Eine Lösung von 46,6 g 6-Amino-6-desoxy-L-sorbofuranose-hydrochlorid-monohydrat wird in 200 ml 0,5 n Salzsäure mit 14,7 g Natriumcyanid versetzt und 3 Stunden gerührt. Anschließend wird bei 25 °C im Vakuum bis zu einem dünnen Sirup eingeengt, mit 200 ml Methanol/Äthanol (1 : 1) versetzt und vom ausgeschiedenen Salz filtriert. Das Filtrat wird bei 25 °C im Vakuum eingeengt, der erhaltene kristalline Feststoff wird mit Äthanol verrührt, abgesaugt und mit Äthanol und Diäthyläther gewaschen. Man erhält 34,5 g (92 % der Theorie) farblose kristalle vom Schmelzpunkt 156 °C (Zers.).

Rf-Wert = 0,194 (Fließmittel 1)
Rf-Wert = 0,119 (Fließmittel 2)
Rf-Wert = 0,6 (Fließmittel 3)

Fließmittel 1 = Chloroform/Methanol/25 %iger Ammoniak im Volumenverhältnis 6 : 4 : 1
Fließmittel 2 = Chloroform/Essigester/Methanol/25 %iger Ammoniaklösung im Volumenverhältnis 40 : 40 : 30 : 1
Fließmittel 3 = Essigester/Methanol/Wasser/25 %ige Ammoniaklösung im Volumenverhältnis 120 : 70 : 10 : 2

## Beispiel 2

2-Amino-methyl-2-hydroxymethyl-3,4,5-trihydroxypiperidin

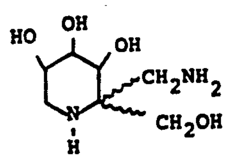

15 g 2,6-Imino-2-hydroxymethyl-2,6-didesoxy-L-ido (L-gulo)-hexonsäurenitril werden in 200 ml Wasser gelöst und nach Zugabe von Raney-Nickel bei 3,5 bar katalytisch hydriert. Der Katalysator wird abfiltriert und die Lösung bei 25 °C im Vakuum eingeengt. Der Eindampfrückstand wird mit 300 ml Methanol bei 40 °C verrührt und nach Zugabe eines Filterhilfsmittels filtriert. Nach Einengen des hellgelben Filtrats erhält man 11,4 g (75 % der Theorie) der gewünschten Verbindungen in Form eines gelblichen Schaumes.

Rf-Wert = 0,34 (DC-Fertigplatten Merck mit Kieselgel 60 F 254, Fließmittel : Methanol/Chloroform/25 %iger Ammoniak 90 : 60 : 60).

## Beispiel 3

2-Benzoylaminomethyl-2-hydroxymethyl-3,4,5-trihydroxypiperidin

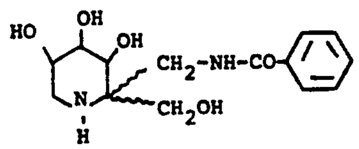

2,0 g 2-Aminomethyl-2-hydroxymethyl-3,4,5-trihydroxypiperidin werden in 20 ml Methanol/Wasser (1 : 1) gelöst, mit 1,45 ml Triäthylamin versetzt und auf – 10 °C abgekühlt. Nach Zugabe von 1,635 g Benzoylchlorid wird 30 Minuten bei – 10 °C und anschließend 20 Stunden bei Raumtemperatur gerührt. Die Lösung wird im Vakuum eingedampft und der Rückstand in 40 ml Methanol/Wasser (2 : 1) aufgenommen. Diese Lösung wird auf eine 40 cm lange und 3 cm weite Säule aufgetragen, die mit einem Kationenaustauscher in der $H^+$-Form gefüllt ist. Es wird zuerst mit 1 l Methanol/Wasser-Gemisch (2 : 1) gewaschen und dann mit 0,1 %igem Ammoniak in Methanol/Wasser (2 : 1) eluiert. Die einzelnen Fraktionen werden dünnschichtchromatographisch auf ihren Gehalt an der gesuchten Verbindung hin untersucht. Die Fraktionen mit dem gewünschten Produkt werden zusammengefaßt und eingedampft. Der Rückstand wird in wenig Methanol gelöst und stehengelassen, wobei bald Kristallisation eintritt. Es wird mit etwas Isopropanol verdünnt, abgesaugt und mit Isopropanol und Diäthyläther gewaschen. Man erhält 0,8 g 2-Benzoylamino-2-hydroxymethyl-3,4,5-trihydroxypiperidin vom Schmelzpunkt 154 bis 156 °C.

Rf-Wert = 0,374 (Fließmittel 1)

Beispiel 4

2-(4-Nitrobenzoylaminomethyl)-2-hydroxymethyl-3,4,5-trihydroxypiperidin

5,4 g 2-Aminomethyl-2-hydroxymethyl-3,4,5-trihydroxypiperidin werden in 50 ml Methanol/Wasser-Gemisch (1 : 1) gelöst, mit 3,92 ml Triäthylamin und bei – 5 °C mit 5,44 g 4-Nitrobenzoylchlorid versetzt. Die Mischung wird 24 Stunden gerührt, im Vakuum eingedampft und in Äther/Wasser aufgenommen und getrennt. Die wäßrige Phase wird einmal mit Äther extrahiert und anschließend eingedampft. Der Rückstand wird in wenig Wasser gelöst und auf eine 120 cm lange und 4 cm weite Säule aufgetragen, die als stationäre Phase Zellulose und als mobile Phase 99 %iges Aceton enthält. Es wird nacheinander mit 99 %igem, 95 %igem und schließlich 90 %igem Aceton eluiert. Die einzelnen Fraktionen werden dünnschichtchromatographisch auf ihren Gehalt an der gesuchten Verbindung hin untersucht. Die Fraktionen mit dem gewünschten Produkt werden zusammengefaßt und eingedampft. Man erhält 2,6 g 2-(4-Nitrobenzoylaminomethyl)-2-hydroxymethyl-3,4,5-trihydroxypiperidin.

Rf-Wert = 0,19 (DC-Fertigplatten Kieselgel 60, Laufmittel : Chloroform/Essigester/Methanol/25 %iger Ammoniak 80 : 80 : 80 : 2).

Rf-Wert = 0,383 (Fließmittel 1)

Beispiel 5

Analog Beispiel 4 wurde 2-(4-methoxybenzoylaminomethyl)-2-hydroxymethyl-3,4,5-trihydroxypiperidin hergestellt.

Rf-Wert = 0,407 (Fließmittel 1)

Beispiel 6

Analog Beispiel 4 wurde 2-(4-Chlorbenzoylaminomethyl)-2-hydroxymethyl-3,4,5-trihydroxypiperidin hergestellt.

Rf-Wert = 0,402 (Fließmittel 1)

Beispiel 7

2-(4-Toluolsulfamidomethyl)-2-hydroxymethyl-3,4,5-trihydroxypiperidin

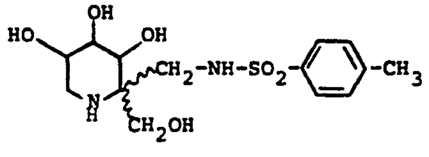

3,7 g 2-Aminomethyl-2-hydroxymethyl-3,4,5-trihydroxypiperidin wurden in 40 ml Methanol/Wasser (1 : 1) gelöst und bei 0 °C mit 4,2 g 4-Toluolsulfochlorid versetzt. Es wurde 30 Minuten bei 0 °C, 1 Stunde bei 20 °C und 30 Minuten bei 50 °C gerührt. Es wurde mit 30 ml Wasser verdünnt und 2 mal mit

Diäthyläther extrahiert. Die wäßrige Phase wurde mit 3 ml 25 %igem Ammoniak versetzt, eingedampft, der Rückstand in wenig Wasser gelöst und auf eine 100 cm lange und 3 cm breite Säule gegeben, die als stationäre Phase Cellulose und als mobile Phase n-Butanol enthielt. Es wurde nacheinander mit n-Butanol, 97,5 %ig und n-Butanol und 95 %igem n-Butanol eluiert. Die einzelnen Fraktionen wurden dünnschichtchromatographisch auf ihren Gehalt an der gewünschten Verbindung hin untersucht. Die Fraktionen, die die gewünschte Verbindung enthielten wurden vereinigt und eingedampft. Man erhielt 1,6 g eines gelblichen Schaumes mit Rf-Wert von 0,28 (DC-Fertigplatten Kieselgel 60 ; Laufmittel : Chloroform/Essigester/Methanol 25 %iger Ammoniak 80 : 80 : 80 : 2).

Rf = 0,43 (Fließmittel 1)

Beispiel 8

2-Acetylaminomethyl-2-hydroxymethyl-3,4,5-trihydroxypiperidin

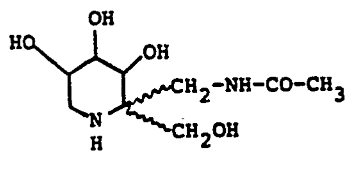

Eine Lösung von 6,5 g rohem 2-Aminomethyl-2-hydroxymethyl-3,4,5-trihydroxypiperidin in 65 ml Methanol/Wasser (1 : 1) wird bei 0 °C mit 5,1 ml Essigsäureanhydrid versetzt. Es wird 30 Minuten bei 0 °C und 18 Stunden bei Raumtemperatur gerührt. Die Lösung wird im Vakuum eingeengt, der Rückstand in wenig Wasser gelöst, zur Entfernung der entstandenen Essigsäure durch einen Anionenaustauscher in der $OH^{\ominus}$-Form filtriert und mit Wasser gewaschen. Das Filtrat wird eingeengt, der Rückstand in wenig Wasser gelöst und auf eine 120 cm lange und 3 cm weite Zellulose-Säule aufgetragen, die als mobile Phase Butanol enthält. Es wird mit Butanol, 95 %igem und 90 %igem Butanol eluiert und die einzelnen Fraktionen dünnschichtchromatographisch auf ihren Gehalt an der gesuchten Verbindung hin untersucht. Die Fraktionen mit dem gewünschten Produkt werden zusammengefaßt und eingeengt. Der Rückstand wird in heißem Methanol gelöst, die Lösung wird filtriert und bis auf ein Volumen von 30 ml eingeengt. Das Produkt kristallisiert aus. Die Kristallisation wird durch Stehen über Nacht vervollständigt. Es wird abgesaugt und mit Methanol gewaschen. Man erhält 2,7 g farblose Kristalle vom Schmelzpunkt 196 °C (Zers.).

Beispiel 9

1-Methyl-2-benzoylaminomethyl-2-hydroxymethyl-3,4,5-trihydroxypiperidin

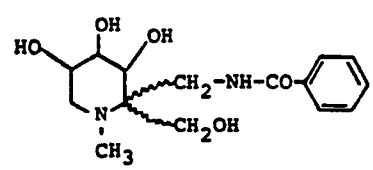

3,95 g 2-Benzoylaminomethyl-2-hydroxymethyl-3,4,5-trihydroxy-piperidin werden in 40 ml absolutem Methanol gelöst und nacheinander mit 3,24 ml Essigsäure und 7,8 ml wäßriger 35 %iger Formaldehydlösung versetzt. Man kühlt auf 0 °C ab und gibt 1,36 g Natriumcyanoborhydrid zu. Man läßt langsam auf Raumtemperatur erwärmen und rührt über Nacht.

Die Lösung wird eingeengt, der Rückstand in 40 ml Methanol/Wasser (2 : 1) gelöst und auf eine 40 cm lange und 3 cm weite Säule aufgetragen, die einen Kationenaustauscher in der $H^+$-Form enthält. Es wird mit ca 1 l Methanol/Wasser (2 : 1) und anschließend mit 0,5 %igem Ammoniak in Methanol/Wasser (2 : 1) gewaschten.

Die einzelnen Fraktionen werden dünnschichtchromatographisch untersucht, die Fraktionen mit dem gesuchten Produkt Zusammengefaßt und eingeengt. Der Eindampfrückstand wird in absolutem Methanol gelöst, die Lösung nach Zugabe eines Filterhilfsmittels filtriert und eingeengt. Man erhält 2,8 g eines gelblichen Schaumes mit einen Rf-Wert von 0,2 (DC-Fertigplatten Kieselgel 60 ; Laufmittel : Chloroform/Essigester/Methanol/25 %iger Ammoniak 80 : 80 : 80 : 2).

Rf-Wert = 0,565 (Fließmittel 1) ; 0,159 (Fließmittel 2).

Beispiel 10

1-Äthyl-2-benzoylaminomethyl-2-hydroxymethyl-3,4,5-trihydroxy-piperidin mit einem Rf-Wert von 0,42

(DC-Fertigplatten Kieselgel 60; Laufmittel: Chloroform/Essigester/Methanol/25 %iger Ammoniak 80 : 80 : 80 : 2).

Rf = 0,636 (Fließmittel 1)

Beispiel 11

2-Aminomethyl-2-hydroxymethyl-3,4,5-trihydroxypiperidin-dihydrochlorid

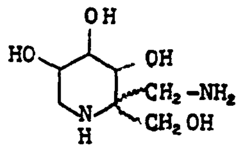

.2 H Cl

15 g 2,6-Imino-2-hydroxymethyl-2,6-didesoxy-L-ido (L-gulo)-hexonsäurenitril (Beispiel 1) werden in 200 ml Wasser in Gegenwart von Raney-Nickel bei 3,5 bar Katalytisch hydriert. Der katalysator wird abfiltriert, die Lösung mit 80 ml 1 n Salzsäure versetzt und eingeengt. Der erhaltene Rückstand kristallisiert beim Einengen mit Methanol. Es wird mit Methanol versetzt, abgesaugt und mit Methanol gewaschen. Man erhält 12,4 g 2-Aminomethyl-2-hydroxymethyl-3,4,5-trihydroxy-piperidin-dihydrochlorid vom Schmelzpunkt 245 °C unter Zersetzung.

Beispiel 12

2-($N^1$-Phenylureidomethyl)-2-hydroxymethyl-3,4,5-trihydroxy-piperidin

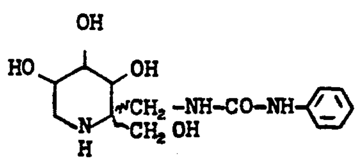

7,95 g (30 m Mol) 2-Aminomethyl-2-hydroxymethyl-3,4,5-trihydroxy-piperidin-dihydrochlorid (Beispiel 11) werden in einem Gemisch von 60 ml (60 m Mol) 1 n Kalilauge und 60 ml Methanol gelöst und bei − 10 °C tropfenweise mit 4,9 ml (42 m Mol) Phenylisocyanat versetzt. Es wird 5 Stdn. bei 20 °C gerührt, mit weiteren 2 ml phenylisocyanat versetzt und 20 Stdn. gerührt. Es wird mit 50 ml Wasser verdünnt und 2x mit Ether extrahiert. Die wäßrige Phase wird eingeengt, der Eindampfrückstand in wenig Methanol aufgenommen und auf eine 120 cm lange und 4 cm weite Säule aufgetragen, die als stationäre Phase Zellulose und als mobile Phase Aceton enthält. Es wird nacheinander mit Aceton, 95 %igem und schließlich 90 %igem Aceton eluiert. Die einzelnen Fraktionen werden dünnschichtchromatographisch auf ihren Gehalt an gesuchter Verbindung hin untersucht. Die Fraktionen mit dem gewünschten Produkt werden zusammengefaßt und eingedampft. Man erhält 5,8 g 2-($N^1$-Phenylureidomethyl)-2-hydroxymethyl-3,4,5-trihydroxy-piperidin als farblosen Schaum.

Rf-Wert = 0,294 (Fließmittel 1)

Massenspektrum: Die wichtigsten peaks im oberen Massenbereich sind: m/e = 280; m/e = 186; m/e = 162.

Beispiel 13

2-(N'-Allyl-thioureidomethyl)-2-hydroxymethyl-3,4,5-trihydroxy-piperidin

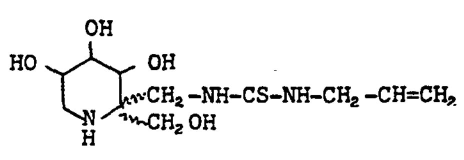

2,65 g (10 m Mol) 2-Aminomethyl-2-hydroxymethyl-3,4,5-trihydroxy-piperidin-dihydrochlorid (Beispiel 11) werden in einer Mischung von 20 ml 1 N Natronlauge und 40 ml Methanol gelöst und unter Eiskühlung tropfenweise mit 2,0 ml Allylsenföl in 20 ml Essigester versetzt. Es wird 18 Stdn. bei Raumtemperatur gerührt und eingeengt. Der Eindampfrückstand wird mit wenig Methanol verrührt; es wird vom unlöslichen Salz abfiltriert und auf eine 120 cm lange und 4 cm weite Säule aufgetragen, die als stationäre Phase Zellulose und als mobile Phase Aceton enthält. Es wird mit Aceton und anschließend mit wäßriger Acetonlösung, deren Gehalt an Wasser gesteigert wird, eluiert. Die einzelnen Fraktionen werden

dünnschichtchromatographisch überprüft. Die Fraktionen mit dem gewünschten Produkt werden zusammengefaßt und eingeengt. Man erhält 1,5 g 2-(N-Allyl-thioureidomethyl)-2-hydroxymethyl-3,4,5-trihydroxypiperidin als nahezu farbloses amorphes Festprodukt.

Massenspektrum : Die wichtigsten peaks im oberen Massenbereich sind : m/e = 260 ; m/e = 234 ; m/e = 203 ; m/e = 162

Rf-Wert = 0,343 (Fließmittel 1) ; 0,483 (Fließmittel 3)

Beispiel 14

N-Hydroxyäthyl-2-benzoylaminomethyl-2-hydroxymethyl-3,4,5-trihydroxypiperidin

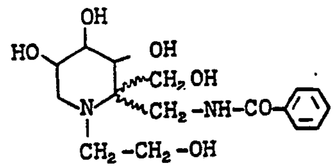

4,44 g 2-Benzoylaminomethyl-2-hydroxymethyl-3,4,5-trihydroxipiperidin (Herstellungsbeispiel 3) werden in 65 ml Wasser gelöst und nach Zugabe eines Tropfens Essigsäure bei ca. 5 °C mit 6 ml Ethylenoxid versetzt. Die Reaktion ist nach 24-stündigem Rühren beendet. Es wird eingeengt, in ca. 40 ml Methanol/Wasser 2 : 1 gelöst und auf eine 20 cm lange und 3 cm weite Säule aufgetragen, die Amberlite® IR 120 $H^+$-Form enthält. Es wird gut mit Methanol/Wasser 2 : 1 gewaschen und anschließend mit 0,1 % Ammoniak eluiert. Die einzelnen Fraktionen werden dünnschichtchromatographisch auf ihren Gehalt hin untersucht, die Fraktionen mit dem gewünschten Produkt werden eingeengt. Man erhält 2,4 g N-Hydroxyethyl-2-benzoylaminomethyl-2-hydroxymethyl-3,4,5-trihydroxypiperidin in Form eines festen Schaumes.

Massenspektrum : Die wichtigsten peaks im oberen Massenbereich sind : m/e = 309 ; m/e = 291 ; m/e = 206 ; m/e = 188

Rf-Wert = 0,50 (Fließmitel 1)

Beispiel 15

N-Nonyl-2-benzoylaminomethyl-2-hydroxymethyl-3,4,5-trihydroxypiperidin

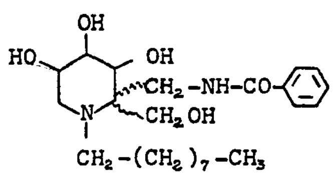

2,96 g 2-Benzoylaminomethyl-2-hydroxymethyl-3,4,5-trihydroxypiperidin (Beispiel 3) werden in einem Gemisch von 40 ml Methanol, 2 ml Wasser und 2,7 ml Essigsäure gelöst und mit 5,7 ml Nonyaldehyd versetzt. Die Lösung wird im Eisbad auf 5 °C abgekühlt ; dann werden 1,35 g Natriumcyanoborhydrid zugefügt. Es wird 30 Minuten unter Kühlung und anschließend 24 Stdn. bei Raumtemperatur gerührt. Es wird eingeengt, in 30 ml Methanol/Wasser 6 : 1 gelöst und auf eine 30 cm lange und 3 cm weite Säule aufgetragen, die mit Amberlite IR 120 $H^+$-Form gefüllt ist. Es wird gut mit Methanol/Wasser im Verhältnis 8 : 1 gewaschen und anschließend mit 0,2 %igem Ammoniak in Methanol/Wasser 6 : 1 eluiert. Die durch Dünnschichtchromatographie ermittelten reinen Fraktionen werden vereint und eingeengt. Man erhält 2,6 g eines nahezu farblosen Oeles.

Massenspektrum : Die wichtigsten peaks im oberen Massenbereich sind : m/e = 391 ; m/e = 373 ; m/e = 288 ; m/e = 228.

Rf-Wert = 0,516 (Fließmittel 2)

Beispiel 16

2-(2-Nitro-benzolsulfamidomethyl)-2-hydroxymethyl-3,4,5-trihydroxypiperidin

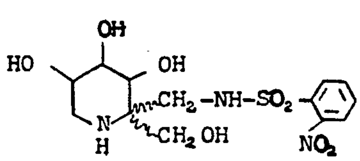

3,3 g 2-Aminomethyl-2-hydroxymethyl-3,4,5-trihydroxypiperidin-dihydrochlorid (Herstellungsbeispiel 11) werden in 150 ml Dimethylformamid mit 14 g kaliumcarbonat 30 Minuten verrührt. Es wird auf

0 °C abgekühlt und mit 6,6 g 2-Nitrobenzolsulfonsäurechlorid versetzt. Es wird 24 Stdn. gerührt, abgesaugt und mit DMF gewaschen. Das Filtrat wird eingeengt, der Eindampfrückstand in Wasser/Essigester verteilt und getrennt. Die wäßrige Phase wird über eine Säule, die Amberlite IR 120 $H^+$-Form enthält, nach den vorangehend beschriebenen Verfahren gereinigt. Man erhält 2,6 g 2-(2-Nitrobenzolsulfamidomethyl)-2-hydroxymethyl-3,4,5-trihydroxypiperidin in Form eines gelblichen Schaumes.

Massenspektrum : Die wichtigsten peaks im oberen Massenbereich sind : m/e = 346 ; m/e = 186 ; m/e = 162.

Rf-Wert = 0,34 (Fließmittel 1)

## Beispiel 17

N-Methyl-2-dimethylaminomethyl-2-hydroxymethyl-3,4,5-trihydroxypiperidin

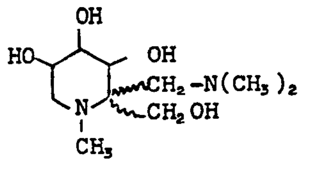

2,65 g 2-Aminomethyl-2-hydroxymethyl-3,4,5-trihydroxypiperidin-dihydrochlorid (Herstellungsbeispiel 11) werden in einem Gemisch von 30 ml Methanol, 16 ml 35 %iger Formaldehydlösung und 3,6 ml Essigsäure gelöst und bei 0-5 °C mit 3,3 g Natriumcyanoborhydrid versetzt. Es wird 20 Stdn. bei Raumtemperatur gerührt und eingeengt. Der erhaltene Eindampfrückstand wird in Methanol/Wasser im Verhältnis 6 : 1 gelöst und auf eine 30 cm lange und 3 cm weite Säule gegeben, die Amberlite IR 120 $H^+$-Form enthält. Es wird mit ca. 2 l Methanol/Wasser im Verhältnis 6 : 1 gewaschen und anschließend mit 0,2 %igem Ammoniak in Methanol/Wasser 6 : 1 eluiert. Die einzelnen Fraktionen werden dünnchichtchromatographisch auf ihren Gehalt untersucht. Die Fraktionen mit dem gewünschten Produkt werden vereint und eingeengt. Das erhaltene Produkt kristallisiert aus wenig Aceton aus. Man erhält 1.4 g farblose Kristalle vom Schmelzpunkt 76 °C

## Beispiel 18

2-(Cyclododecylaminomethyl)-2-hydroxymethyl-3,4,5-trihydroxypiperidin

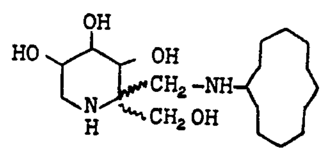

2,65 g 2-Aminomethyl-2-hydroxymethyl-3,4,5-trihydroxypiperidin-dihydrochlorid (Herstellungsbeispiel 11) werden in 21 ml Wasser gelöst und anschließend mit 60 ml Methanol, 1,5 ml Essigsäure und 6 g Cyclododecanon versetzt. Nach erfolgter Lösung werden 1,35 g Natriumcyanoborhydrid zugegeben. Es wird 18 Stdn. gerührt, das ausgefallene Produkt wird abgesaugt und mit Wasser gewaschen. Man erhält 2,4 g eines farblosen Komplexes, der zur Freisetzung der Base auf eine Amberlite IR 120 $H^+$-Austauschsäule aufgetragen wird. Es wird mit ca. 3 l Wasser gewaschen und die gewünschte Verbindung mit 2 %igem Ammoniakwasser freigesetzt. Die durch Dünnschichtchromatographie ermittelten Fraktionen des Produktes werden zusammengefaßt und eingeengt. Der Eindampfrückstand kristallisiert beim Verreiben mit Isopropanol. Man erhält 1,8 g eines farblosen Produktes vom Schmelzpunkt 171-172 °C.

## Beispiel 19

2-Hexyloxycarbonylaminomethyl-2-hydroxymethyl-3,4,5-trihydroxypiperidin

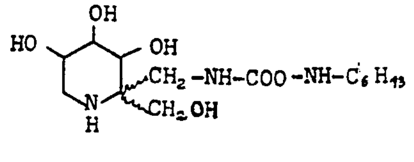

5,3 g 2-Aminomethyl-2-hydroxymethyl-3,4,5-trihydroxypiperidin-dihydrochlorid (Beispiel 11) werden in 20 ml Wasser gelöst und mit 30 ml Methanol und 8,4 ml Triethylamin versetzt. In diese Lösung werden bei 0-5 °C 7,8 ml Chlorameisensäurehexylester, gelöst in 30 ml Essigester, getropft. Nach dreistündigem Rühren bei Raumtemperatur wird eingeengt. Der Eindampfrückstand wird in Wasser/Essigester verteilt,

die Phasen werden getrennt und die Essigesterphase 1x mit Wasser gewaschen. Die vereinten Wasserphasen werden 1 Std. mit 80 g Amberlite IR A-400 $OH^-$-Form verrührt, es wird abgesaugt und eingeengt. Der Eindampfrückstand wird in wenig Methanol gelöst und auf eine 120 cm lange und 4 cm weite Säule aufgetragen, die als stationäre Phase Zellulose und als mobile Phase Aceton enthält. Es wird mit Aceton gewaschen. Die Substanz wird durch Eluieren mit 95 %igem Aceton erhalten. Nach dem Einengen erhält man 2,4 g eines nahezu farblosen gelben Schaumes.

Rf-Wert : 0,542 (Fließmittel 1) ; 0,143 (Fließmittel 2)

Weitere Herstellungsbeispiele sind in den nachfolgenden Tabellen aufgeführt.

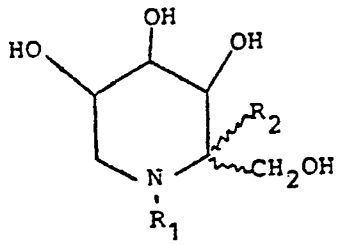

| Beispiel-Nr. | $R_1$ | $R_2$ | analog Beispiel Nr. | wichtige Massenpeaks m/e | Rf-Wert (Fließmittel) |
|---|---|---|---|---|---|
| 20 | H | $-CH_2-NH-CO-C_6H_{13}$ | 3 | 273<br>162 | 0,453 (1) |
| 21 | $-_nC_{11}H_{23}$ | $-CH_2-NH-CO-$ 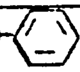 | 15 | 419<br>316<br>286 | 0,524 (2) |
| 22 | $-_nC_4H_9$ | $-CH_2-NH-CO-$ 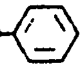 | 15 | 321<br>218 | 0,421 (2) |
| 23 | $CH_3$ | $-CH_2-NH-CO-nC_6H_{13}$ | 9 | 267<br>269<br>176 | 0,607 (1) |
| 24 | $-_nC_9H_{19}$ | $-CH_2-NH-CO-nC_6H_{13}$ | 15 | 399<br>283 | 0,879 (1) |
| 25 | $n-C_7H_{15}$ | $-CH_2-NH-CO-$ 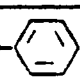 | 15 | 363<br>260<br>230 | 0,484 (2), |
| 26 | $-C_3H_7$ | $-CH_2-NH-CO-$ 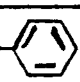 | 15 | 307<br>204 | 0,389 (2), |
| 27 | H | $-CH_2-NH-CO-C_8H_{17}$ | 3 | 314<br>301<br>162 | 0,495 (1) |
| 28 | H | $-CH_2-NH-CH(CH_3)-(CH_2)_3-N(C_2H_5)_2$ | 18 | 333<br>258<br>243<br>162 | 0,415 |

| Bei-spiel-Nr. | $R_1$ | $R_2$ | analog Her-stellungs-beispiel | Die wich-tigsten Massen-peaks m/e | Rf-Wert (Fließ-mittel) |
|---|---|---|---|---|---|
| 29 | H | 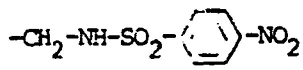 | 16 | 346<br>202<br>162 | 0,34 (1) |
| 30 | H | 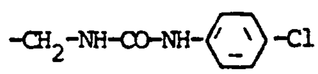 | 12 | 235<br>187<br>162 | 0,302 (1)<br>0,402 (3) |
| 31 | H | $-CH_2-NH-CO-NH-CH_3$ | 12 | 218<br>187<br>162 | 0,146 (1)<br>0,119 (3) |
| 32 | H | $-CH_2-NH-CO-NH-C_2H_5$ | 12 | 232<br>187<br>162 | 0,198 (1)<br>0,184 (3) |
| 33 | H | 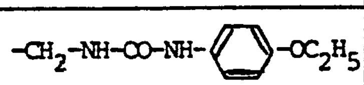 | 12 | 187<br>163<br>163 | 0,292 (1)<br>0,352 (3) |
| 34 | H | 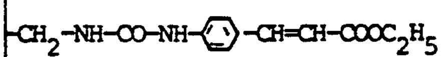 | 12 | 217<br>191<br>187<br>162 | 0,368 (1)<br>0,419 (3) |
| 35 | H | 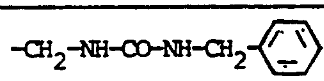 | 12 | 187<br>162<br>106 | 0,307 (1)<br>0,328 (3) |
| 36 | H | 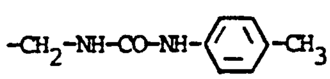 | 12 | 187<br>162<br>133 | 0,311 (1)<br>0,377 (3) |
| 37 | H | 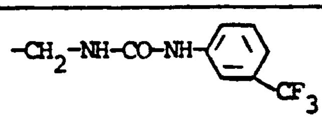 | 12 | 187<br>162<br>161 | 0,321 (1) |

| Bei-spiel-Nr. | $R_1$ | $R_2$ | analog Herstellungs-beispiel | Die wichtigsten Massen-peaks m/e | Rf-Wert (Fließ-mittel) |
|---|---|---|---|---|---|
| 38 | H | $-CH_2-NH-CO-NH-C_{12}H_{25}$ | 13 | 187<br>169<br>162 | |
| 39 | H | $-CH_2-NH-CS-NH-$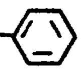 | 12 | 234<br>203<br>162<br>93 | 0,454 (1) |
| 40 | H | $-CH_2-NH-CS-NH-CH_3$ | 12 | 234<br>203<br>162 | 0,222 (1)<br>0,308 (3) |
| 41 | H | $-CH_2-NH-CO-NH-$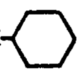 | 12 | 286<br>187<br>162<br>99 | 0,352 (1) |
| 42 | H | $-CH_2-NH-CO-NH-$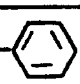$-NO_2$ | 13 | Schmp.:<br>206°C | |
| 43 | H | $-CH_2-NH-CO-NH-CH_2-CH=CH_2$ | 13 | 244<br>187<br>162 | 0,241 (1)<br>0,217 (3) |
| 44 | $CH_3$ | $-CH_2-NH-CO-NH-$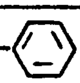 | 9 | 201<br>176<br>119 | 0,426 (1) |
| 45 | H | $-CH_2-NH-CO-NH-C_4H_9$ | 13 | | 0,275 (3) |
| 46 | H | $-CH_2-NH-CO-NH-$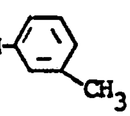 | 13 | 187<br>162<br>133 | 0,315 (1) |
| 47 | $CH_3$ | $-CH_2-NH-CS-NH-$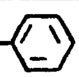 | 9 | | 0,692 (3) |

| Beispiel-Nr. | $R_1$ | $R_2$ | analog Herstellungsbeispiel | Die wichtigsten Massenpeaks m/e | Rf-Wert (Fließmittel) |
|---|---|---|---|---|---|
| 48 | H | $-CH_2-NH-CO-NH-$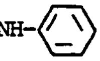$-F$ | 13 | 311<br>280<br>187<br>162 | 0,287 (1)<br>0,392 (1) |
| 49. | H | $-CH_2-NH-CO-CH_2-OCH_3$ | 13 | | 0,148 (1) |
| 50 | $C_6H_{13}$ | $-CH_2-N(C_6H_{13})_2$ | 17 | | 0,825 (2) |
| 51 | H | $-CH_2-NH-CO-NH-(CH_2)_5-CN$ | 13 | | 0,308 (1) |
| 52 | H | $-CH_2-NH-CO-NH_2$ | 13 mit Cyansäure | | 0,0695 (1) |
| 53 | H | $-CH_2-NH-CO-NH-(CH_2)_2-COOiC_4H_9$ | 13 | | 0,417 (1) |
| 54 | H | $-CH_2-NH-COOCH_3$ | 19 | | 0,278 (1) |

**Ansprüche**

1. Verbindungen der allgemeinen Formel

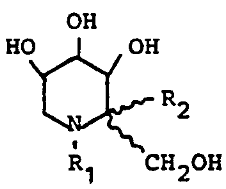

(I)

worin

$R_1$ Wasserstoff oder einen gegebenenfalls durch Halogen, Hydroxy oder $C_1$-$C_4$-Alkoxy substituierten geradkettigen, verzweigten, gesättigten oder ungesättigten aliphatischen Kohlenwasserstoffrest mit bis zu 18 C-Atomen darstellt und

$R_2$ $-CH_2NR_3R_4$, $-CH_2-NR_3-CO-R_4$, $-CH_2-NR_3-CONR_4R_5$, $-CH_2-NR_3-SO_2R_4$, $-CH_2-NR_3-SO_2-NR_4-R_5$, $-CH_2-NR_3-CS-R_4$, $-CH_2-NR_3-CS-NR_4R_5$, $-COOR_3$, $-CO-NR_3R_4$, $-CH_2-OR_3$, $-CH_2-NH-COOR_4$ oder $-CH_2-NH-COSR_4$,

$R_3$, $R_4$ und $R_5$ Wasserstoff, einen gegebenenfalls durch Hydroxy, $C_1$-$C_4$-Alkoxy, $C_1$-$C_4$-Alkoxycarbonyl, Carboxy, Amino, Di-$C_1$-$C_4$-Alkylamino oder Cyan substituierten aliphatischen Kohlenwasserstoffrest mit bis zu 18 C-Atomen, eine $C_5$-$C_{12}$-Cycloalkylgruppe, Benzyl oder einen Phenylrest der gegebenenfalls durch $C_1$-$C_4$-Alkyl, $C_1$-$C_4$-Alkoxy, Halogen, Nitro, $CF_3$; Carboxy, Alkoxycarbonyl, Amino oder den $-CH=CH-COOC_2H_5$-Rest substituiert ist,

bedeuten.

2. Verfahren zur Herstellung der Verbindungen gemäß Anspruch 1, dadurch gekennzeichnet, daß man 2,6-Imino-2-hydroxymethyl-2,6-didesoxy-L-ido (L-gulo)-hexonsäurenitril der Formel III

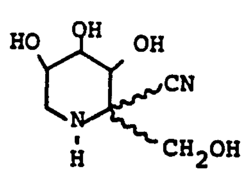

(III)

entweder

a) katalytisch zur Verbindung der Formel IV

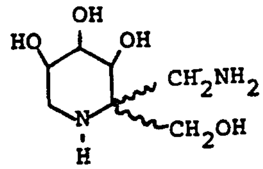

(IV)

hydriert und anschließend gegebenenfalls die primäre Aminogruppe alkyliert, acyliert, sulfonyliert oder mit Isocyanaten oder Isothiocyanaten umsetzt und anschließend gegebenenfalls am sekundären Stickstoffatom alkyliert oder

b) die Verbindung der Formel III zur Verbindung der Formel V

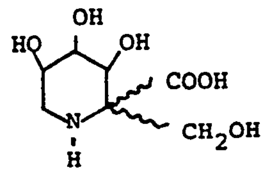

(V)

verseift und anschließend gegebenenfalls verestert und die Ester gegebenenfalls entweder mit Aminen zu Amiden umsetzt oder gegebenenfalls mit Wasserstoff-Donor-Reduktionsmitteln zu Verbindungen der Formel VI

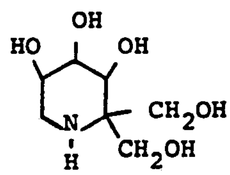

(VI)

reduziert und alle auf diesem Wege entstandenen Verbindungen gegebenenfalls am sekundären Stickstoffatom alkyliert, oder indem man Verbindungen der Formel VII

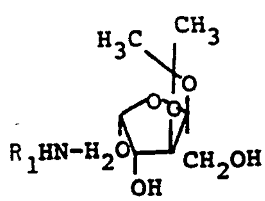

(VII)

worin $R_1$ die in Anspruch 1 angegebene Bedeutung hat mit Mineralsäuren zu Verbindungen der Formel VIII

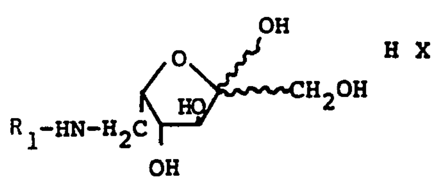

(VIII)

deblockiert, diese dann direkt in Lösung oder nach Isolierung mit Cyaniden zu Verbindungen der Formel IX

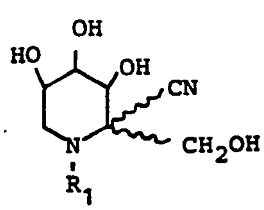

(IX)

umsetzt und gegebenenfalls am sekundären Stickstoffatom alkyliert.

3. Arzneimittel, gekennzeichnet durch einen Gehalt an einer Verbindung gemäß Anspruch 1 und gegebenenfalls pharmazeutisch geeigneten Zusatzstoffen.

4. Tierfuttermittel, gekennzeichnet durch einen Gehalt an einer Verbindung gemäß Anspruch 1.

**Claims**

1. Compounds of the general formula

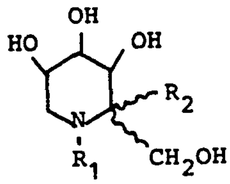

(I)

wherein

$R_1$ represents hydrogen or a straight-chain, branched, saturated or unsaturated aliphatic hydrocarbon radical with up to 18 carbon atoms which is optionally substituted by halogen, hydroxy or $C_1$-$C_4$-alkoxy and

$R_2$ $-CH_2NR_3R_4$, $-CH_2-NR_3-CO-R_4$, $-CH_2-NR_3-CONR_4R_5$, $-CH_2-NR_3-SO_2R_4$, $-CH_2-NR_3-SO_2-NR_4R_5$, $-CH_2-NR_3-CS-R_4$, $-CH_2-NR_3-CS-NR_4R_5$, $-COOR_3$, $-CO-NR_3R_4$, $-CH_2-OR_3$, $-CH_2-NR_3-COOR_4$ or $-CH_2-NR_3-COSR_4$,

$R_3$, $R_4$ and $R_5$ denote hydrogen, an aliphatic hydrocarbon radical with up to 18 carbon atoms which is optionally substituted by hydroxy, $C_1$-$C_4$-alkoxy, $C_1$-$C_4$-alkoxy-carbonyl, carboxyl, amino, di-$C_1$-$C_4$-alkylamino or cyano, a $C_5C_{12}$-cycloalkyl group, benzyl or a phenyl radical which is optionally substituted by $C_1$-$C_4$-alkyl, $C_1$-$C_4$-alkoxy, halogen, nitro, $CF_3$, carboxyl, alkoxycarbonyl, amino or the $-CH=CH-COOC_2H_5$ radical.

2. Process for the preparation of the compounds according to Claim 1, characterised in that 2,6-imino-2-hydroxymethyl-2,6-didesoxy-L-ido (L-gulo)-hexonic acid nitrile of the formula III

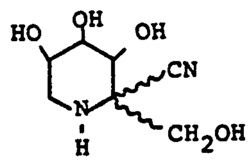

(III)

is either

a) catalytically hydrogenated to give the compound of the formula IV

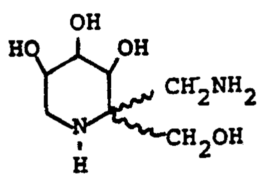

(IV)

and the primary amino group is then optionally alkylated, acylated, sulphonylated or reacted with isocyanates or isothiocyanates, and the secondary nitrogen atom is then optionally alkylated, or

b) the compound of the formula III is saponified to give the compound of the formula V

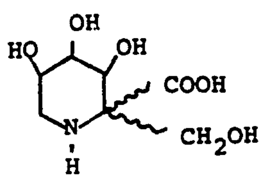

(V)

and this compound is then optionally esterified and the esters are either optionally reacted with amines to give amides or optionally reduced with hydrogen donor reducing agents to give compounds of the formula VI

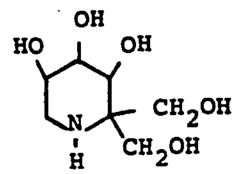

(VI)

and all the compounds formed in this way are optionally alkylated on the secondary nitrogen atom, or by a process in which compounds of the formula VII

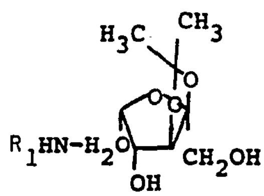

(VII)

wherein $R_1$ has the meaning given in Claim 1, are deblocked with mineral acids to give compounds of the formula VIII

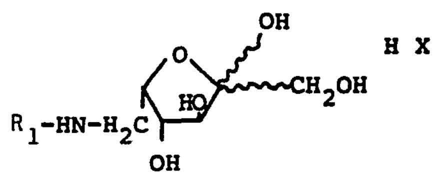

(VIII)

these compounds are then reacted directly in solution, or after isolation, with cyanides to give compounds of the formula IX

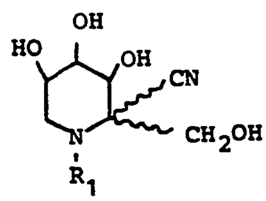

(IX)

and the secondary nitrogen atom is optionally alkylated.

3. Medicaments, characterised in that they contain a compound according to Claim 1 and, if appropriate, pharmaceutically suitable additives.

4. Animal feedstuff, characterised in that it contains a compound according to Claim 1.

**Revendications**

1. Composés de formule générale

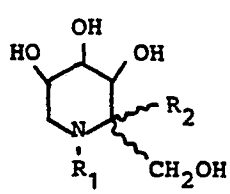

(I)

dans laquelle

$R_1$ représente l'hydrogène ou un reste d'hydrocarbure aliphatique saturé ou insaturé à chaîne droite ou ramifiée jusqu'en $C_{18}$, éventuellement substitué par un reste halogéno, hydroxy ou alcoxy en $C_1$-$C_4$ et

$R_2$ représente $-CH_2NR_3R_4$, $-CH_2-NR_3-CO-R_4$, $-CH_2-NR_3-CONR_4R_5$, $-CH_2-NR_3-SO_2R_4$, $-CH_2-NR_3-SO_2-NR_4-R_5$, $-CH_2-NR_3-CS-R_4$, $-CH_2-NR_3-CS-NR_4R_5$, $-COOR_3$, $-CO-NR_3R_4$, $-CH_2-OR_3$, $-CH_2-NH-COOR_4$ ou $-CH_2-NH-COSR_4$,

$R_3$, $R_4$ et $R_5$ représentent l'hydrogène, un reste d'hydrocarbure aliphatique jusqu'en $C_{18}$ éventuellement substitué par des restes hydroxy, alcoxy en $C_1$-$C_4$, (alcoxy en $C_1$-$C_4$) carbonyle, carboxy, amino, di(alkyl en $C_1$-$C_4$) amino ou cyano, un groupe cycloalkyle en $C_5$-$C_{12}$, un reste benzyle ou un reste phényle

qui est éventuellement substitué par un reste alkyle en $C_1$-$C_4$, alcoxy en $C_1$-$C_4$, halogéno, nitro, $CF_3$ ; carboxy, alcoxycarbonyle, amino ou le reste —CH = CH—$COOC_2H_5$.

2. Procédé pour la préparation des composés selon la revendication 1, caractérisé en ce que, ou bien

a) on hydrogène catalytiquement le 2,6-imino-2-hydroxyméthyl-2,6-didésoxy-L-ido (L-gulo) hexononitrile de formule III

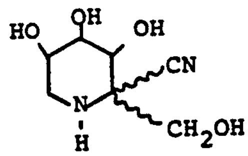

(III)

en le composé de formule IV

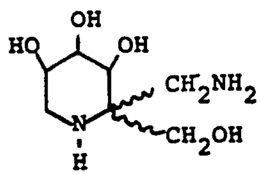

(IV)

et ensuite on alkyle, on acyle, on sulfonyle éventuellement le groupe amino primaire ou on le fait réagir avec des isocyanates ou des isothiocyanates et ensuite on l'alkyle éventuellement à l'atome d'azote secondaire, ou bien

b) on saponifie le composé de formule III en le composé de formule V

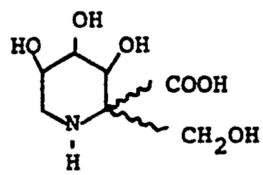

(V)

et ensuite on estérifie éventuellement et, ou bien on fait éventuellement réagir les esters avec des amines en amides, ou bien on les réduit éventuellement avec des agents réducteurs donneurs d'hydrogène en composés de formule VI

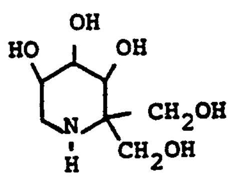

(VI)

et on alkyle éventuellement à l'atome d'azote secondaire tous les composés se formant de cette manière, ou bien on débloque les composés de formule VII

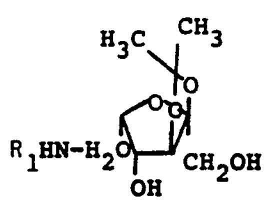

(VII)

dans laquelle $R_1$ a la signification indiquée à la revendication 1, avec des acides minéraux en composés de formule VIII

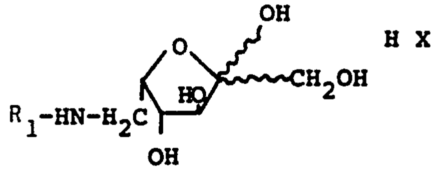

(VIII)

on fait ensuite réagir ceux-ci directement en solution ou après isolement avec des cyanures en composés de formule IX

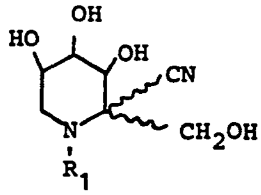

(IX)

et éventuellement on les alkyle à l'atome d'azote secondaire.

3. Médicaments, caractérisés en ce qu'ils contiennent un composé selon la revendication 1, et éventuellement des additifs appropriés en pharmacie.

4. Aliments pour animaux, caractérisés en ce qu'ils contiennent un composé selon la revendication 1.